# EUROPEAN PATENT APPLICATION

(11) **EP 4 563 090 A1**
(43) Date of publication of application: **04.06.2025**
(21) Application number: 24216335.0
(22) Date of filing: 29.11.2024
(51) Int. Cl.: A61B 8/08, A61B 8/00, A61B 8/12

(54) **GUIDED ENTRANCE TO EPICARDIAL SACK WITH TRANSTHORACIC ULTRASOUND**

(30) Priority: 30.11.2023 US 202318524174
(71) Applicant: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL)
(74) Representative: Carpmaels & Ransford LLP

(57) **Abstract**

A method includes using an imaging system external to a body of a patient, the imaging system fitted with a first position sensor. An image is acquired, of an anatomical landmark of an organ of the patient, while a position and an orientation of the imaging system are tracked using a positioning system. The anatomical landmark is marked on the image. The location of the mark is computed in a 3D coordinate system of the positioning system. The computed location is stored in a memory.

## Description

### FIELD OF THE DISCLOSURE

The present disclosure relates generally to guiding invasive medical probes, and particularly to guiding a transthoracically inserted magnetically-position-tracked needle further using an ultrasound reference image.

### BACKGROUND OF THE DISCLOSURE

Techniques to assist in guiding an invasive medical probe were previously proposed in the patent literature. For example, U.S. patent 7,918,793 describes how an image of an electro-anatomical map of a body structure having cyclical motion is overlaid on a 3D ultrasonic image of the structure. The electro-anatomical data and anatomic image data are synchronized by gating both electro-anatomical data acquisition and an anatomic image at a specific point in the motion cycle. Transfer of image data includes identification of a point in the motion cycle at which the 3-dimensional image was captured or is to be displayed.

As another example, U.S. patent 9,414,770 describes a method including positioning body-electrodes in galvanic contact with a body of a patient and locating a probe in the body of the patient. Positions of the probe are tracked during respiration of the patient and indications are determined that are related to impedances between the body-electrodes during the respiration. The method also includes calculating a function relating the positions of the probe to the indications and applying the function to identify end-expirium points of the respiration based on Subsequent indications related to the impedances.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Fig. 2 is a schematic, pictorial illustration of a combined ultrasound, magnetic tracking, and electrical tracking system configured for motion-compensated guiding of a transthoracically inserted needle and catheter, in accordance with an example of the present disclosure;
Fig. 3 is a flow chart that schematically illustrates a method for guiding a needle inserted transthoracically, in accordance with an example of the present disclosure; and
Fig. 4 is a flow chart that schematically illustrates a method for motion-compensated guidance of the needle inserted transthoracically in Fig. 3, in accordance with an example of the present disclosure.

### DETAILED DESCRIPTION OF EXAMPLES

### OVERVIEW

Pericardial treatment, such as radiofrequency (RF) ablation and/or pulsed field ablation (PFA), may be used to alleviate heart problems, such as certain ventricular arrhythmia (VA) types. The VA can be treated by catheter ablation, where the catheter is inserted transthoracically via a needle. However, it is difficult to guide a physician to insert the needle through the thorax and through the pericardial sac at the apex, as the exact location of the apex is not fully known during the procedure (e.g., due to different patients' postures). The apex location is also sensitive to the breathing cycle. One possible way to resolve this is the use of fluoroscopy which involves applying high doses of X-ray radiation.

Examples of the present disclosure that are described hereinafter provide a technique to indicate to the physician a direct line of sight to the apex during transthoracic insertion of a position-tracked needle, and followingly, the insertion of a catheter via the needle, to treat the apex tissue. This is done without the need for fluoroscopy while inserting the catheter into the pericardial sac.

The technique includes acquiring a reference image of the thorax and the heart using an imaging system fitted with a first position sensor (e.g., a magnetic tree axial sensor (TAS)). The US transducer is external to the body of a patient. The US imaging is performed within the working volume of a positioning system, (e.g., CARTO^{®} with a location pad placed below the patient). The user acquires a US image of an anatomical landmark of an organ of the patient (e.g., of the LV apex), while the positioning system tracks the position and the angle at which the US transducer is being held based on signals received from the TAS. The position and orientation of the handheld US transducer are determined in the coordinate system of the positioning system.

In some examples, the imaging system is a handled US transducer. In other examples, the imaging system may be single-plane or biplane fluoroscopy imaging system. Although there is exposure related to capturing the reference image with fluoroscopy, the exposure is expected to be significant less than that required to guide the catheter during a procedure.

Once the user locates the apex on the reference image, the user marks the apex location on the image, and this marked location is computed in a 3D coordinate system of the positioning system, and the computed location together with the orientation of the transducer when the apex was observed is used to identify the three-dimensional location of the marked apex within the working volume of a positioning system. This later be used to determine how to insert the needle through the thoracic region to reach the apex of the heart.

To this end, after the physician marks the apex in the US image, the processor computes the location of the mark in a 3D coordinate system of the positioning system and stores that location in space. The processor calculates this location using the known position and orientation of the US handle, and the known field of view of the US image in relation to the US handle. The calculated (i.e., computed) location is stored in a memory.

In a subsequent clinical procedure, a physician transthoracically inserts into the body of the patient a needle fitted with a second position sensor (e.g., a magnetic position sensor). The physician can navigate the needle to the anatomical landmark by using the same or similar positioning system to track in real-time the position of the needle inside the body relative to the stored computed apex location.

In one example, a processor of the positioning system provides a trajectory, e.g., a linear trajectory that aligns needle orientation with the US estimated orientation to the computed location of the LV apex. The disclosed technique provides further guiding of the needle using a visual tool that shows the apex location and the needle's current location marked in the 3D coordinate system of the positioning system.

The disclosed technique renders real-time US imaging during the insertion of the needle unnecessary since the location is already marked. However, insertion through the pericardial sac at the apex remains a challenge due to LV apex location sensitivity to the breathing cycle.

In some examples, the processor monitors the impedances between the patches of an electrical location detection system to estimate the resulting movement of the apex. In one example, several patches are positioned on the chest and three patches positioned on the patient's back. The impedance change between the patches is correlated with an observed movement of the apex and can be used by the processor to motion-correct the marked location of the apex to guide the physician in reaching the apex during its continual cyclic motion.

### SYSTEM DESCRIPTION

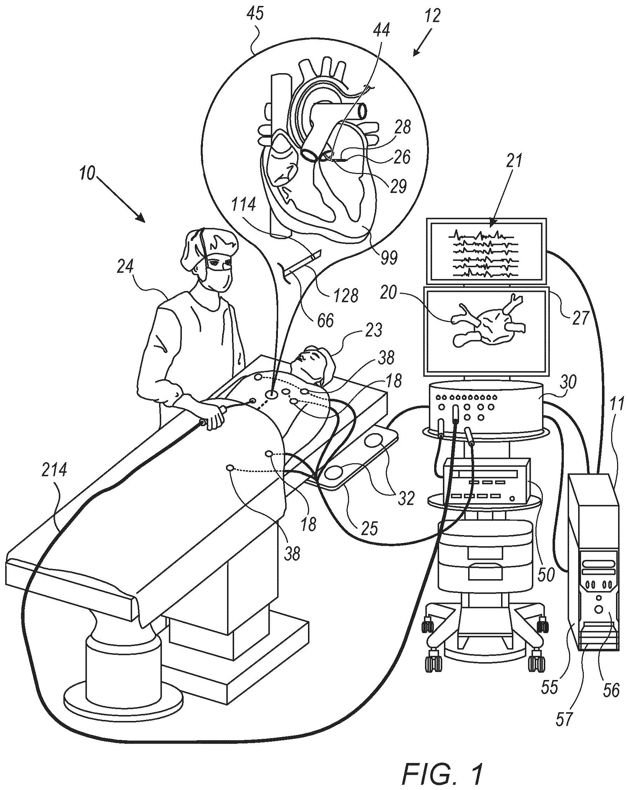

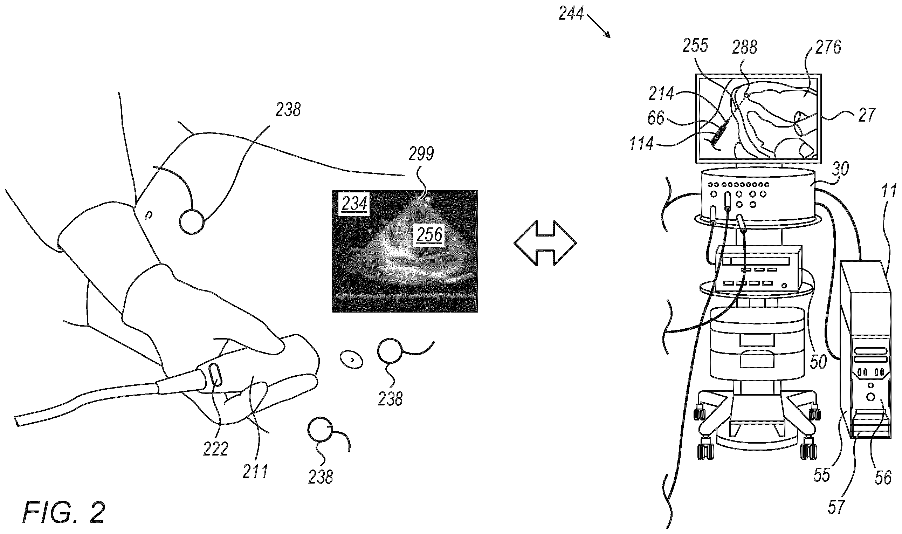

Fig. 1 is a schematic, pictorial illustration of a catheter-based electroanatomical (EA) mapping and ablation system 10, in accordance with an example of the present disclosure. Fig. 1 shows a physician 24 transthoracically inserting a position-tracked needle 66 into the thorax of a patient 23 and subsequently inserting via the needle a catheter 214, as shown in greater detail in Fig. 2.

As seen in inset 45, a magnetic-based position sensor 114 is disposed over a distal end 128 of needle 66. Using a positioning system employing position sensor 114, a processor guides needle 66 (or tube 66) into the vicinity of heart 12, as described below.

Using the transthoracic needle 66 insertion, catheter 214 can be inserted via needle 66 into the thorax of the patient to access a heart 12 to sense and ablate arrhythmogenic epicardial tissue at an LV apex 99, as further shown in Fig. 2.

System 10 may include multiple catheters, where in inset 45 catheter 14 is shown for clarity being inserted separately from needle 66 (but can also be inserted by physician 24 through needle 66). Catheter 214 and catheter 14 may be the same type of catheter.

The plurality of catheters may include catheters dedicated for sensing intracardiac electrograms (IEGM) and/or for both sensing and ablating, as well as imaging catheters. The example tip catheter 14 that is configured for sensing IEGM and to perform electrical ablation is also illustrated herein (in inset 45, physician 24 brings a tip assembly 28, which includes a position sensor 29 and is fitted on a shaft 44 of catheter 14, into contact with the heart wall for sensing and/or ablating a target site in heart 12 using a tip electrode 26).

As noted above, magnetic-based position sensors 114 and 29 are fitted at distal end 128 of needle 66 and distal end 28 of catheter 14, respectively. Sensors 114 and 29 can be operated together with a location pad 25, which includes a plurality of magnetic coils 32 configured to generate magnetic fields in a predefined working volume. Details of this example of a positioning system, i.e., of a magnetic-based position-sensing (i.e., 3D magnetic positioning) technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location references for location pad 25 as well as impedance-based tracking functionality of electrodes 26. For impedance-based tracking, electrical current is directed toward electrodes 26 and sensed at electrode skin patches 38 so that the location of each electrode can be triangulated via electrode patches 38. Details of the impedance-based location-tracking technology are described in US Patent Nos. 7,536,218; 7,756,576; 7,848,787; 7,869,865; and 8,456,182.

Signals from electrode patches 38 are used by an electrical positioning system employing processor 56 to provide breathing motion compensation while guiding catheter 11 to LV apex 99, as further described in Fig. 2.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses as may be used to affect irreversible electroporation (IRE), or combinations thereof.

Patient interface unit (PIU) 30 is an interface configured to establish electrical communication between catheters, electrophysiological equipment, power supply and a workstation 55 for controlling system 10 operation. Electrophysiological equipment of system 10 may include, for example, multiple catheters, location pad 25, body surface ECG electrodes 18, electrode patches 38, ablation energy generator 50, and recorder 11. Optionally and preferably, PIU 30 additionally includes processing capability for implementing real-time computations of catheter locations and for performing ECG calculations.

Workstation 55 includes memory 57, processor 56 unit with memory or storage with appropriate operating software loaded therein, and user interface capability. Workstation 55 may provide multiple functions, optionally including (i) modeling the endocardial anatomy in three-dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered electroanatomical (EA) map 20, (iii) displaying real-time location and orientation of multiple catheters within the heart chamber, and (iv) displaying sites of interest on display device 27, such as places where ablation energy has been applied. One commercial product embodying elements of system 10 is available as the CARTO^{™} 3 System, available from Biosense Webster, Inc., 31 Technology Drive, Suite 200, Irvine, CA 92618.

### MOTION-COMPENSATED GUIDING SYSTEM TO LV APEX TARGET

Fig. 2 is a schematic, pictorial illustration of a combined ultrasound, magnetic tracking, and electrical tracking system configured for motion-compensated guidance of a transthoracically inserted needle 66 and a catheter 214 via needle 66, in accordance with an example of the present disclosure. While the current example shows the ultrasound acquisition and the rest of the system elements together, in general, the US images can be acquired and marked (e.g., tagged) separately (e.g., at an earlier time and in a different location) than that of the catheter-guiding procedure. This is possible since needle guidance relies only on location-marked US images, and the location information of the US handheld transducer is acquired during the US procedure, and not during real-time US acquisition.

Fig. 2 shows an imaging system such as a handheld US transducer 211 fitted with a magnetic position sensor 222 (e.g., TAS 222), that emits signals indicative of position and orientation that are acquired by a positioning system (the same magnetic position tracking system such as those included in system 10 of Fig. 1 used in guiding needle 66) .

The US system generates a US image 234 of the heart, which includes LV 256 and LV apex 299. The location and orientation of the handheld US transducer 211 are analyzed and recorded by processor 56 after the user marked LV apex 299 on at least one of the US images.

Alternatively, other imaging systems such as a single-plane or bi-plane fluoroscopy imaging system is used and a position sensor is mounted on the imaging portion of the system.

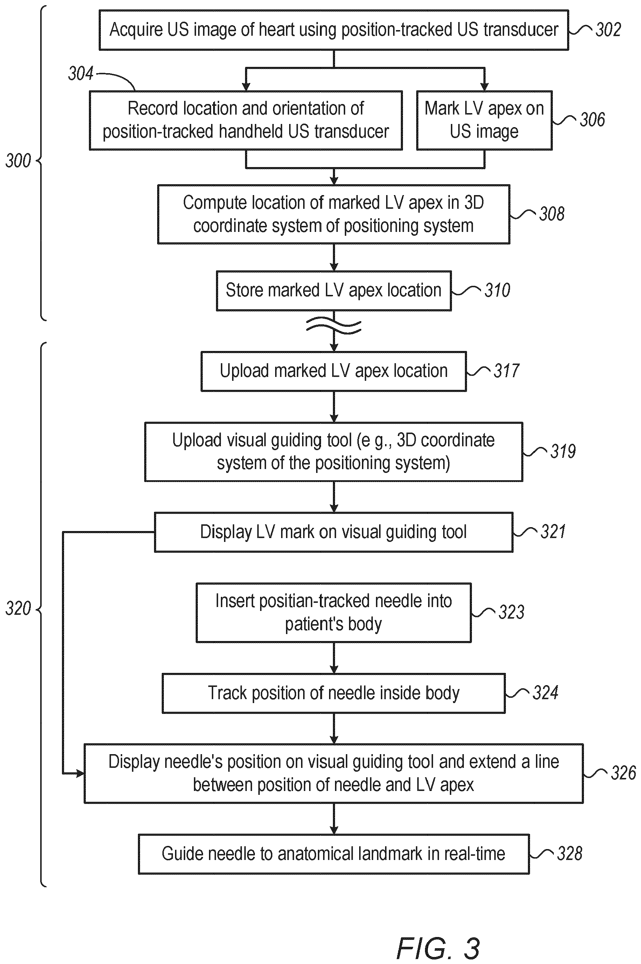

As described in the flow chart of Fig. 3 below, processor 56 displays (27) a respectively (to 299) computed LV mark 288 on an anatomical model 244 of LV 276 using stored position information of LV mark 299. Processor 56 further displays needle 66 on the anatomical model and adds a straight (i.e., linear) trajectory 255 to the computed LV apex mark 288. Alternatively, the processor can display linear trajectory 255 between the needle position and the marked anatomical location in 3D the coordinate system of the positioning system.

Once needle 66 was advanced to place, the physician inserted catheter 214 (e.g., a catheter like catheter 14) via needle 66 to diagnose and/or treat the LV apex tissue.

### MOTION-COMPENSATED METHOD OF GUIDING A CATHETER TO LV APEX TARGET

Fig. 3 is a flow chart that schematically illustrates a method for guiding a transthoracically inserted probe, in accordance with an example of the present disclosure. The method includes two phases: US tracking phase 300 and a subsequent catheter tracking phase 320. These phases can be performed in this order at different times and different locations.

The algorithm, according to the presented example, carries out a process that begins in phase 300 with a US operator, such as physician 24, performing a noninvasive US scan of heart 12 using handheld US transducer 211 at US imaging step 302. Handheld US transducer 211 includes a TAS sensor and the imaging is performed within the working volume of your positioning system, e.g., CARTO^{®}.

In parallel, a processor records the location and orientation of the handheld US transducer 211, at a handheld US transducer tracking step 304. The location and orientation are determined in the coordinate system of the positioning system.

In a tagging step 306, the physician marks (299) the LV apex on the US image 234 of heart LV area 256.

In landmark location computing step 308, after the physician marks the apex in the US image, the processor computes the location of the mark in the 3D coordinate system of the positioning system. The processor calculates this location using the known location and orientation of the handle, and the known field of view of the US image in relation to the handle. The relationship may be determined for example during a calibration procedure.

In data storing step 310, the user stores that location.

The process described by phase 320 begins at a data uploading step 317, when a user, such as the physician 24, uploads the LV apex location calculated and stored during steps 308-310 above to system 10.

Next, at a visual guiding tool operating step, the user, or a processor, opens a visual guiding tool, such as one showing a 3D coordinate system of the positioning system.

Assuming a visual tool was opened, at LV marking displaying step 321, the processor displays the LV mark (that identifies the clinical target for a catheter) on the tool. The LV apex location can be marked there (e.g., as a star), with the current location (of the distal end of the needle also marked (e.g., as a cursor). Additionally, the visual tool can show a line extending between the current location of the needle and the starred location to indicate the path to follow in needle insertion. As the physician inserts the needle, the cursor advances based on its tracked location. The physician may command to change the orientation of the view (e.g., rotate the 3D coordinate system).

At a needle insertion step 322, physician 24 inserts the position-tracked needle into the patient's body (e.g., thorax). The needle includes a TAS sensor preferably at the distal tip of the needle.

At needle position tracking step 324, the processor records the location and orientation of distal end 128 inside the patient's body.

At needle visualization step 326, after the processor has identified the position of the needle, the processor marks it on the screen and extends a line between the location of the needle and the apex.

At a needle guiding step 328, using the position signals from sensor 114 of needle 66, the processor can guide distal end 128, by, for example, calculating and displaying the advancement of distal end 128 superimposed on the anatomical model relative to the LV apex mark (e.g., tag 288) on the anatomical model, at a needle guiding step 328.

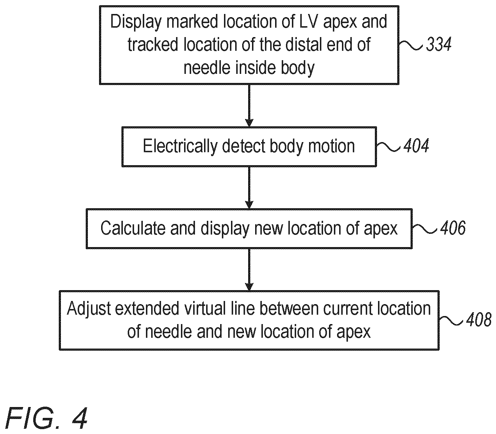

As described in Fig. 4 below, guiding the needle in real-time includes adjusting the trajectory 255 to the LV to compensate for breathing motion.

Once needle 66 is in place, the physician can puncture the sac and then insert a catheter through the puncture.

The flow chart shown in Fig. 3 is chosen purely for the sake of conceptual clarity. The present example may also comprise additional steps of the algorithm, such as occasionally using X-ray imaging. This and other possible steps are omitted from the disclosure herein purposely to provide a more simplified flow chart.

Fig. 4 is a flow chart that schematically illustrates a method for motion compensation during the guiding of the transthoracically inserted needle of Fig. 3, in accordance with an example of the present disclosure.

The process starts with a visualization step 402, where the processor shows on display 27 the location of the apex and the location of the distal end of the needle inside the body.

At an electrical detection of body motion step 404, the processor receives electrical position signals from an electrical location system that employs patches 38 as described in Figs. 1 and 2. These signals are indicative of the LV marking motion with body motion (e.g., with patient breathing).

At Apex location monitoring and adjustment 406, the processor calculates and displays the new location of the apex.

At adjustment step 408, the processor adjusts the extended virtual line 255 based on the current location of the needle and the newly (due to body motion) calculated (i.e., computed) marked location 288 of the apex.

Once the needle is at the target location, the physician inserts via the needle a catheter to perform the intended procedure (e.g., an ablation to eliminate an LV arrhythmia).

### EXAMPLES

### Example 1

A method includes using an imaging system (211) external to a body of a patient, the imaging system (211) fitted with a first position sensor (222). An image (234) is acquired, of an anatomical landmark (99) of an organ (12) of the patient, while a position and an orientation of the imaging system (211) are tracked using a positioning system. The anatomical landmark (99) is marked (299) on the image (234). The location of the mark is computed in a 3D coordinate system of the positioning system. The computed location (288) is stored in a memory (57).

### Example 2

The method according to example 1, and comprising inserting a needle (66) fitted with a second position sensor (114) into the body of the patient. A position of the needle (66) is tracked inside the body. Using the positioning system and the location, a user is guided to navigate the needle to the anatomical landmark (99).

### Example 3

The method according to any of examples 1 and 2, wherein guiding the needle (66) to the anatomical landmark (99) comprises using a visual tool (244) with the location marked on.

### Example 4

The method according to any of examples 1 through 3, wherein the visual tool (244) shows a 3D coordinate system of the positioning system.

### Example 5

The method according to any of examples 1 through 4, wherein guiding further comprises displaying a linear trajectory (255) between the needle (66) position and the computed anatomical location (288) in the 3D coordinate system of the positioning system.

### Example 6

The method according to any of examples 1 through 5, and comprising, using body motion tracking (238), adjusting the marked location (288) to compensate for the body motion.

### Example 7

The method according to any of examples 1 through 6, wherein the first and the second position sensors (222, 114) are magnetic position sensors used by a magnetic positioning system.

### Example 8

The method according to any of examples 1-7, wherein the imaging system is a handheld ultrasound probe.

### Example 9

The method according to any of examples 1-7, wherein the imaging system is a single-plane or bi-plane fluoroscopy imaging system.

### Example 10

A system (10) includes an imaging system (211) and a processor (56). The imaging system (211) is external to a body of a patient, the imaging system (211) fitted with a first position sensor (222) and configured to acquire an image (234) of an anatomical landmark (99) of an organ (12) of the patient while a position and an orientation of the imaging system (211) is tracked, using the first position sensor (222), by a positioning system. The processor (56)is configured to (i) mark (299) the anatomical landmark (99) on the image (234), (ii) compute the location (288) of the mark in a 3D coordinate system of the positioning system, and (iii) store the marked location in a memory (57).

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A system (10), comprising:
an imaging system (211) external to a body of a patient, the imaging system (211) fitted with a first position sensor (222) and configured to acquire an image (234) of an anatomical landmark (99) of an organ (12) of the patient while a position and an orientation of the imaging system (211) is tracked, using the first position sensor (222), by a positioning system; and
a processor (56), which is configured to:
mark (299) the anatomical landmark (99) on the image (234);
compute the location (288) of the mark in a 3D coordinate system of the positioning system; and
store the computed location in a memory (57).

2. The system according to claim 1, and comprising:
a needle configured for insertion into the body of the patient, the needle fitted with a second position sensor; and
a processor that is configured to track a position of the needle inside the body, and, using the positioning system and the location, guide a user to navigate the needle to the anatomical landmark.

3. The system according to claim 1, wherein the processor is configured to guide the needle to the anatomical landmark by using a visual tool with the location marked on.

4. The system according to claim 3, wherein the visual tool shows a 3D coordinate system of the positioning system.

5. The system according to claim 4, wherein the processor is further configured to guide by displaying a linear trajectory between the needle position and the computed anatomical location in the 3D coordinate system of the positioning system.

6. The system according to claim 1, wherein the processor is configured to, using body motion tracking, adjust the computed location to compensate for the body motion.

7. The system according to claim 1, wherein the first and the second position sensors are magnetic position sensors used by a magnetic positioning system.

8. The system according to claim 1, wherein the imaging system is a handheld ultrasound probe.

9. The system according to claim 1, wherein the imaging system is a single-plane or bi-plane fluoroscopy imaging system.
